# EUROPÄISCHE PATENTSCHRIFT

(11) **EP 2 740 514 B1**
(45) Veröffentlichungstag und Bekanntmachung des Hinweises auf die Patenterteilung: **05.04.2017**
(21) Anmeldenummer: 13191854.2
(22) Anmeldetag: 07.11.2013
(51) Int. Cl.: A61N 1/05, A61N 1/372

(54) **Implantierbare Elektrostimulationsanordnung sowie Adapter und Elektrodenleitung einer solchen**
Implantable electrostimulation arrangement and adapter and alectrode lead of the same
Ensemble d'électrostimulation implantable ainsi qu'adaptateur et ligne d'électrode correspondants

(30) Priorität: 04.12.2012 US 201261732950 P
(43) Veröffentlichungstag der Anmeldung: 11.06.2014
(73) Patentinhaber: BIOTRONIK SE & Co. KG, 12359 Berlin (DE)
(72) Erfinder: Dörr, Thomas, 12437 Berlin (DE)
(74) Vertreter: Galander, Marcus

(56) Entgegenhaltungen:
- US-A1- 2003 149 456
- US-A1- 2005 033 370
- US-B2- 6 968 235

## Beschreibung

Die Erfindung betrifft eine implantierbare Elektrostimulationsanordnung, mit einem Elektrostimulationsgerät und einer im Gebrauchszustand an das Elektrostimulationsgerät angeschlossenen Elektrodenleitung. Sie betrifft des Weiteren eine Elektrodenleitung und einen Leitungsadapter einer solchen Anordnung.

Elektrostimulationsanordnungen werden seit langem insbesondere zur Therapie von Herzrhythmusstörungen, aber auch im Rahmen spezieller Therapien anderer Organe, etwa des Gehirns, eingesetzt. Implantierbare Systeme für den Dauer- bzw. Langzeiteinsatz umfassen typischerweise ein an einer bestimmten geeigneten Stelle im Körper platziertes Elektrostimulationsgerät und eine Elektrodenleitung, die die Stimulationsimpulse zum Therapieort leitet. Da es viele verschiedene Typen von Elektrostimulationsgeräten und Elektrodenleitungen - auch von zahlreichen verschiedenen Herstellern - gibt, die im Einsatz wahlweise zu kombinieren sind, müssen für die konkrete Therapie die relevanten Konstruktionsmerkmale und Parameter der Elektrodenleitung bekannt sein. Daher haben Elektrodenleitungen Elektrodenidentifikatoren (Typ, Seriennummer o. ä.), mit deren Hilfe die relevanten Parameter ermittelbar sind und die daher entweder direkt an das Stimulationsgerät oder an eine die Therapie vorbereitende und steuernde Zentrale einer Klinik oder kardiologischen Praxis übermittelt werden.

Ähnlich ist die Situation beim Einsatz von Systemen zur Erfassung von Körperaktionspotentialen, bei denen an die Stelle des Elektrostimulationsgerätes ein mit der Elektrodenleitung verbundenes Abführgerät tritt, sowie bei kombinierten Abführ- und Stimulationsanordnungen. Auch diese gehören zum Gebiet der vorliegenden Erfindung.

Traditionell werden auf die Verpackung einer Elektrodenleitung aufgedruckte Elektrodenidentifikatoren bei der Ingebrauchnahme der Elektrodenleitung von der Verpackung abgelesen und manuell in einen Arbeitscomputer des entsprechenden Systems eingegeben und dort im erforderlichen Umfang verarbeitet, um letztlich den Betrieb des Elektrostimulationsgerätes entsprechend den Parametern der Elektrodenleitung steuern zu können.

Es sind auch Anordnungen bekannt geworden, bei denen die Elektrodenidentifikatoren in Speicherchips innerhalb der Elektrodenleitung elektronisch gespeichert sind; vgl. etwa US 6,968,235 B2. Eine solche zusätzliche elektronische Komponente in der Elektrodenleitung erhöht jedoch die Komplexität von deren Aufbau und hat daher höhere Kosten und jedenfalls tendenziell auch eine höhere Ausfallwahrscheinlichkeit zur Folge. Zudem ist beim implantierbaren Gerät eine Schnittstelle zum Auslesen des Speicherchips erforderlich, so dass bei Weitem nicht sämtliche marktgängigen Geräte mit einer entsprechend ausgestatteten Elektrodenleitung kombinierbar sind.

Der Erfindung liegt daher die Aufgabe der Bereitstellung einer unter Kosten- und Zuverlässigkeitsaspekten verbesserten und weitgehend universell aus marktgängigen Komponenten zu erstellenden Elektrostimulationsanordnung zugrunde. Diese Aufgabe wird durch eine Elektrostimulationsanordnung mit den Merkmalen des Anspruchs 1 gelöst. Des Weiteren werden eine Elektrodenleitung mit den Merkmalen des Anspruchs 11 sowie ein Leitungsadapter mit den Merkmalen des Anspruchs 14 bereitgestellt. Zweckmäßige Fortbildungen des Erfindungsgedankens sind Gegenstand der jeweiligen abhängigen Ansprüche.

Die Erfindung schließt die Überlegung ein, den zwischenzeitlich eingeschlagenen Weg, in den Elektrodenleitungen elektronisch lesbare Elektrodenidentifikatoren zu speichern, wieder zu verlassen und auf jegliches zusätzliche Bauteil in den dauerhaft implantierten Anordnungskomponenten, das zum Zwecke der Elektrodenidentifikation dient, zu verzichten. Sie schließt weiter den Gedanken ein, stattdessen die Elektrodenleitungen (und nicht deren Verpackung) in maschinenlesbarer - insbesondere optisch lesbarer - Weise zu kennzeichnen, was zugleich bedeutet, die Elektrodenidentifikation direkt von der Leitung abzulesen. Weiter gehört zur Erfindung der Gedanke, hierzu ein spezielles Bauteil vorzusehen, welches vor der permanenten Implantation der Anordnung temporär in diese einbezogen, nach Erfüllung seiner Aufgabe aber wieder entfernt wird. Ebenso liegt der Erfindung der Gedanke zu Grunde, während der Nachsorge den Austausch des Elektrostimulationsgerätes vorzunehmen, ohne eine schon implantierte und soeben im Stand der Technik beschriebene Elektrodenleitung ebenfalls auszutauschen und deren Elektrodenidentifikation auszulesen. Schließlich sieht die Erfindung vor, dass zu diesem Zweck ein Leitungsadapter zum Einsetzen zwischen das Elektrostimulationsgerät und die Elektrodenleitung vorgesehen ist, der Lesemittel zum Lesen der Elektrodenidentifikation und Sendemittel zu deren Übermittlung in das Elektrostimulationsgerät und/oder an einen anordnungs-externen Empfänger aufweist.

Da dieser Leitungsadapter nicht zum permanent implantierten System gehört, wird dessen Komplexität durch die vorgeschlagene Lösung nicht erhöht und auch die systemimmanente Ausfallwahrscheinlichkeit nicht beeinflusst. Da im Leitungsadapter elektronische Standard-Bauteile eingesetzt werden können, hält sich die mit seinem Vorsehen verbundene Kostenerhöhung des Systems in Grenzen; je nach konkreter Konfiguration ergibt sich für die Elektrodenleitungen ggf. überhaupt keine Kostensteigerung. Kostensseitig vorteilhaft wirkt sich aber die Zeiteinsparung und Vermeidung von Fehlerquellen durch den Fortfall menschlicher Erkennungs- und Eingabetätigkeiten bei der Implantationsvorbereitung aus.

In einer Ausführung der Erfindung ist der Leitungsadapter im Lieferzustand zusammen mit dem Elektrostimulationsgerät in einer gemeinsamen sterilen Verpackung angeordnet. Alternativ hierzu kann vorgesehen sein, dass der Leitungsadapter im Lieferzustand zusammen mit der Elektrodenleitung in einer gemeinsamen sterilen Verpackung angeordnet ist. Eine weitere Variante besteht darin, dass der Leitungsadapter im Lieferzustand einzeln verpackt und insbesondere re-sterilisierbar ausgeführt ist.

Bevorzugt ist die Elektrodenidentifikation aus der Elektrodenleitung mechanisch, optisch und/oder elektrisch, insbesondere elektromagnetisch oder mittels Hochfrequenz auslesbar. Dementsprechend weist dann der Leitungsadapters Lesemittel auf, die mechanische, optische und/oder elektrische Aufnahmemittel, insbesondere elektromagnetische Ausnahmemittel oder Hochfrequenz aufnehmende Mittel aufweisen.

Mechanische Aufnahmemittel erfassen dabei Elektrodenidentifikationen, die über eine spezielle Form oder Oberflächengestaltung des Elektrodensteckers realisiert sind, wie z.B. eine Kodierung über Einkerbungen im Stecker.
Optische Aufnahmemittel erfassen optische Merkmale des Elektrodensteckers oder der Elektrodenleitung oder ein optisch lesbaren Identifikator, wie z.B. einen Barcode. Eingesetzt werden hier z.B. einen Kamera, eine CCD-Zeile oder ähnliche optische Aufnahmegeräte.
Elektrische Aufnahmemittel erfassen über eine galvanische Verbindung mit der Elektrodenleitung, vorzugsweise über den ohnehin vorhandenen Steckerkontakt, in die Elektrode als Identifikator integrierte elektrische Eigenschaften, wie z.B. das Auslesen einer digitalen Kennung in einem Identifikationschip im Elektrodenstecker. Dies kann beispielsweise aber auch eine charakteristische elektrische Größe sein, wie beispielsweise eine charakteristische Leitungsimpedanz, -kapazität oder ähnliches.
Elektromagnetische und Hochfrequenz-Aufnahmemittel erfassen über eine kontaktfreie Verbindung mit der Elektrodenleitung in die Elektrode als Identifikator integrierte elektrische Eigenschaften, wie z.B. das Auslesen einer digitalen Kennung in einem Identifikationschip im Elektrodenstecker, so z.B. das Auslesen einer RF-ID oder eines vergleichbaren Transponderchips, wobei die Stromversorgung des Identifikationschips über die elektromagnetische bzw. Hochfrequenzenergie erfolgt.

In einer weiter bevorzugten Ausführung ist die Elektrodenidentifikation durch die Lesemittel des Leitungsadapters optisch berührungslos und/oder elektrisch galvanisch oder berührungslos auslesbar.
Im letzteren Fall sind besonders bevorzugte Ausführungen möglich, bei denen die optisch lesbare Elektrodenidentifikation einen Barcode oder eine Buchstaben/Ziffern-Kombination aufweist und die Lesemittel des Leitungsadapters einen Barcode-Scanner oder einen Text-Scanner umfassen. Wenn das System auf Barcode-Basis aufgebaut wird, kann gleichwohl eine Buchstaben/Ziffern-Kombination zusätzlich zum Barcode an der Elektrodenleitung vorgesehen sein, um erforderlichenfalls eine unmittelbare Überprüfung durch das mit der Zusammenfüllung des Systems betraute medizinische Personal zu ermöglichen.

Eine weitere Ausführung besteht in der Einbringung einer RF-ID in den Elektrodenstecker, die von der Aufnahmeeinheit im Leitungsadapter berührungslos mittels eines RF-ID-Leseverfahrens abgefragt wird.

Vorzugsweise ist die optisch lesbare Elektrodenidentifikation in oder an einem Stecker der Elektrodenleitung vorgesehen. Abweichend hiervon kann aber auch ein separater Marker bzw. ein Tag an die eigentliche Leitung angespritzt oder in sonstiger Weise angefügt sein, der/das den Elektrodenidentifikator trägt.

Gemäß einer weiteren Ausführung der Erfindung weisen die Sendemittel des Leitungsadapters eine Funkschnittstelle zur telemetrischen Übertragung der Elektrodenidentifikationsdaten in das Elektrostimulationsgerät oder zur drahtlosen Übermittlung an eine korrespondierende Funkschnittstelle eines Therapiesystems auf. Die konkrete technische Ausgestaltung der Sendemittel richtet sich nach dem bevorzugten Modus - Telemetrie-Übertragung in das implantierbare Gerät (IMD) oder etwa WLAN-Übertragung zu einem Eingabe-Computer des Kliniksystems, o. ä. - und wird von konzeptionellen Überlegungen des Systemherstellers bestimmt sein. Die aufgrund der jeweiligen Entscheidung zu bestimmende Spezifikation der Funk-Schnittstelle liegt im Rahmen fachmännischen Handelns und bedarf daher hier keiner weiteren Beschreibung.

In einer weiteren Ausführung ist vorgesehen, dass der Leitungsadapter einen Elektrodenidentifikations-Umsetzer zur Umkodierung der gelesenen Elektrodenidentifikation gemäß Anschluss-Spezifikationen des Elektrostimulationsgeräts und zur Übergabe der umkodierten Elektrodenidentifikation an die Sendemittel zur Übermittlung in das Elektrostimulationsgerät aufweist. Diese Ausführung wird insbesondere bei einer Bereitstellung des Adapters in Kombination mit dem IMD relevant, da der Elektrodenidentifikations-Umsetzer mit Parmametern des IMD zu programmieren ist. In einer Ausgestaltung der letztgenannten Ausführung sind Sendemittel zur Übermittlung der Elektrodenidentifikation an den anordnungs-externen Empfänger, also einen Empfänger, der sich außerhalb der Anordnung befindet, zur Übersendung sowohl der gelesenen Elektrodenidentifikation als auch der umkodierten Elektrodenidentifikationsdaten ausgebildet. In der Therapiezentrale werden also nicht nur die entsprechend dem konkreten IMD umkodierten Daten, sondern zusätzlich auch die ursprünglichen Elektrodenidentifikationsdaten verfügbar gemacht.

Vorteile und Zweckmäßigkeiten der Erfindung ergeben sich im Übrigen aus der nachfolgenden Beschreibung von Ausführungsbeispielen und - aspekten anhand der Figuren. Von diesen zeigen:
- Fig. 1A und 1B: Prinzipskizzen einer Ausführungsform der Erfindung (temporärer Zwischenzustand bzw. permanenter Implantationszustand),
- Fig. 2: eine perspektivische Darstellung einer Ausführungsform der Erfindung und
- Fig. 3: eine schematische Darstellung des Aufbaus eines beispielhaften Leitungsadapters in Art eines Funktions-Blockschaltbildes.

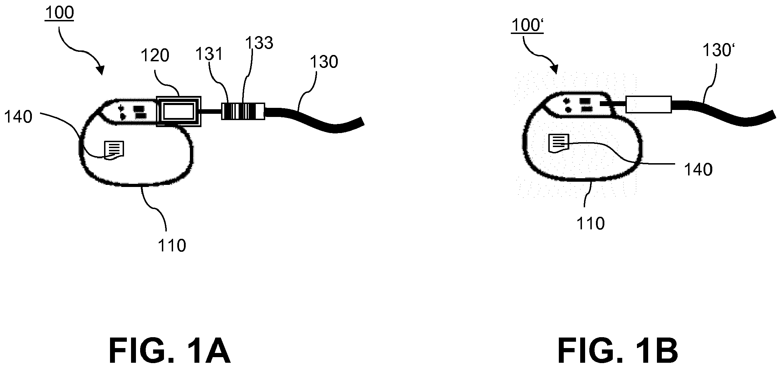

Fig. 1A zeigt schematisch eine implantierbare Elektrostimulationsanordnung 100, deren Stimulationsgerät hier ein Herzschrittmacher 110 ist. An den Herzschrittmacher 110 ist über einen Leitungsadapter 120 eine Elektrodenleitung 130 angeschlossen. Die Elektrodenleitung 130 hat einen Anschlussstecker 131, an dessen Außenoberfläche ein Barcode 133 angebracht ist, in dem die Elektrodenidentifikationsdaten der Elektrodenleitung kodiert sind. Der Leitungsadapter 120 ist zum Lesen dieses Barcodes 133 und zur Übermittlung entsprechend umkodierter Daten über eine Telemetrie-Schnittstelle an die Steuerung des Schrittmachers 110 ausgebildet, wo diese zur Schrittmachereinstellung für die nachfolgende dauerhafte Implantation verarbeitet und in einem Elektrodendatenspeicher 140 gespeichert werden.

Vor der Implantation der Stimulationsanordnung wird der Leitungsadapter 120 wieder entfernt und die Elektrodenleitung 130 dauerhaft an den Schrittmacher 110 angeschlossen, so dass im Prinzip der in Fig. 1B gezeigte Zustand der Anordnung entsteht. Fig. 1B dient jedoch, indem sie eine andere Elektrodenleitung 130' zeigt, speziell zur Darstellung einer weiteren System-Option: Wenn das Elektrostimulationsgerät (der Schrittmacher) nämlich nach einer initialen Einstellung auf eine bestimmte Elektrodenleitung anhand von deren Elektrodenidentifikationsdaten letztlich mit einer anderen Elektrodenleitung (hier bezeichnet mit Ziffer 130') verbunden wird, deren Elektrodenidentifikationsdaten nicht auf die vorliegend beschrieben Weise in die Steuerung und den Speicher 140 des Schrittmacher übertragen werden können, so gelten die dort vorab verarbeiteten und gespeicherten Daten so lange weiter, solange nicht eine externe Umprogrammierung über eine telemetrisch angeschlossenes Programmiergerät ausgeführt wird. Auch mit Elektrodenleitungen, die nicht erfindungsgemäß ausgestaltet sind, ist somit durchaus eine Arbeits- und Zeitersparnis bei der Implantation erreichbar (wenn nämlich deren wesentliche Daten mit denjenigen einer vorab "eingelesenen" Elektrodenleitung übereinstimmen), wobei in jedem Fall eine Eingabe der Daten von nicht erfindungskonform ausgestalteten Elektrodenleitungen in das Elektrostimulationsgerät auf herkömmliche Weise erfolgen kann.

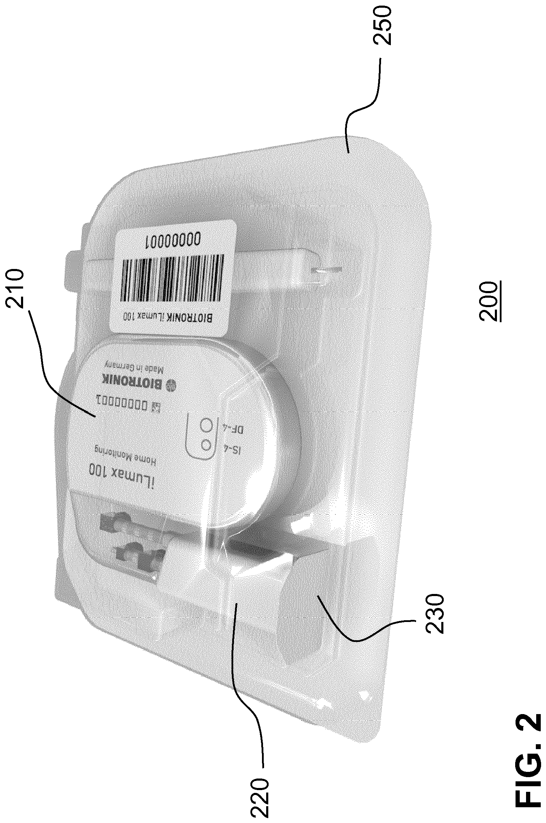

Fig. 2 zeigt eine Verpackungseinheit 200 eines Herzschrittmachers 210, der zusammen mit einem Leitungsadapter 220, der mit einer stabilen Schutzfolie 230 versehen ist, in einer Sterilverpackung 250 (sog. "Innenblister", der zur Auslieferung und zum Transport noch eine Umverpackung hat) untergebracht ist. Die Verpackungseinheit 200 kann im sterilen Klinikbereich eingesetzt werden, und der Leitungsadapter 220 ist im Anschlussbereich des Schrittmachers 210 vormontiert, so dass (nicht gezeigte) Elektrodenleitungen ohne Werkzeug nach Entfernung der Schutzfolie 230 in den Adapter eingeführt werden und die vom Leitungsadapter im Zusammenwirken mit dem Schrittmacher auszuführenden Lese-, Mess- und Übertragungsvorgänge selbsttätig ausgelöst werden können.

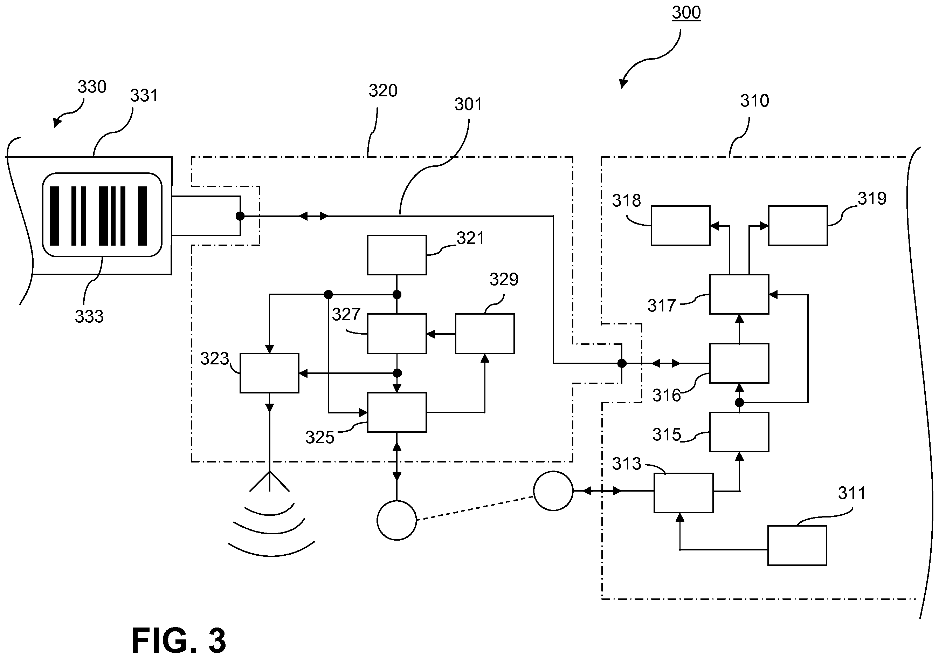

Fig. 3 zeigt in Art eines Funktions-Blockschaltbildes wesentliche Funktionskomponenten einer Herzschrittmacheranordnung 300 im Zustand der Implantationsvorbereitung, in dem zwischen einen Herzschrittmacher 310 und eine Elektrodenleitung 330, ein Leitungsadapter 320 geschaltet ist. Vom Herzschrittmacher 310 sind hier nur die im Zusammenhang mit der Erfindung wesentlichen Komponenten/Funktionen dargestellt und erläutert; ansonsten kann es sich um einen praktisch beliebigen herkömmlichen Schrittmacheraufbau handeln.

An einem Stecker 131 der Elektrodenleitung 330 ist ein Barcode 333 aufgebracht, der durch einen Barcode-Scanner 321 des Leitungsadapters 320 gelesen wird. Der Barcode-Scanner 321 ist ausgangsseitig einerseits mit einer Elektrodenidentifikations-Sendestufe 323 verbunden, die über eine (nicht gesondert bezeichnete) Antenne die durch den Barcode repräsentierte Elektrodenidentifikation der Elektrodenleitung 330 beispielsweise an einen bei der Vorbereitung der Implantation eingesetzten Arbeitsplatzcomputer eines Therapiesystems per WLAN übermittelt.

Andererseits ist der Barcode-Scanner 321 ausgangsseitig mit einer Telemetrie-Sende/Empfangsstufe 325 und einer Umkodiereinheit 327 verbunden. Die Telemetrie-Sende/Empfangsstufe 325 dient einerseits zur telemetrischen Übermittlung der Elektrodenidentifikation über eine (wiederum nicht gesondert bezeichnete) Sendespule an den Schrittmacher 310 und andererseits zum Empfang von Schrittmacherdaten, die in einen Schrittmacherdatenspeicher 329 des Leitungsadapters 320 gelangen. Unter Nutzung dieser Daten erfolgt eine schrittmacherspezifische Umkodierung der Elektrodenidentifikation in der Umkodierstufe 327, und auch diese umkodierten Daten werden an die Telemetrie-Sende/Empfangsstufe 325 zur Übertragung an den Schrittmacher übergeben. Es versteht sich, dass die Funktionalität der Umkodierung der Elektrodenidentifikation unter Berücksichtigung von Schrittmacherdaten auch im Schrittmacher selbst vorgesehen sein könnte; ihre Realisierung im Leitungsadapter 320 ermöglicht es aber, über die Funksendestufe 323 auch die umkodierten Daten während der Initialisierungs-Prozedur direkt an das Therapiesystem der Klinik oder kardiologischen Praxis zu übermitteln.

Im Schrittmacher sind ein Schrittmacherdatenspeicher 311 und eine schrittmacherseitige Telemetrie-Sende/Empfangsstufe 313 zur Bereitstellung der Schrittmacherdaten und zu deren Übermittlung an den Leitungsadapter 320 sowie zum Empfang der Elektrodenidentifikationsdaten und umkodierten Elektrodenidentifikationsdaten vom Leitungsadapter 320 vorgesehen. Die beiden Sätze von Elektrodenidentifikationsdaten gelangen von der Telemetrie-Sende/Empfangsstufe 313 in einen Elektrodendatenspeicher 315 und werden in einer Elektrodenmessstufe 316 zum Ausmessen der Elektrodenleitung über die temporär über den Leitungsadapter 320 hergestellte Leitungsverbindung 301 genutzt. Die Messergebnisse gelangen von der Elektrodenmessstufe 316 über eine Verarbeitungsstufe 317 in einen Programmspeicher 318 und einen Arbeitsspeicher 319 des Schrittmachers 310 und werden dem Schrittmacherbetrieb nach Abschluss der Initialisierungsphase zu Grunde gelegt. In den Arbeitsspeicher 319 gelangen, soweit benötigt, auch die Ergebnisse einer direkten Verarbeitung der aus dem Elektrodendatenspeicher 315 in die Verarbeitungseinheit 317 geladenen Elektrodendaten.

## Patentansprüche

1. Implantierbare Elektrostimulationsanordnung (100; 300), mit einem Elektrostimulationsgerät (110; 210; 310), einem Leitungsadapter (120; 220; 320) und einer im Gebrauchszustand an das Elektrostimulationsgerät angeschlossenen Elektrodenleitung (130; 330),
wobei die Elektrodenleitung eine auslesbare Elektrodenidentifikation (133; 333) aufweist,
**dadurch gekennzeichnet, dass**
der Leitungsadapter (120; 220; 320) zum temporären Einsetzen zwischen das Elektrostimulationsgerät und die Elektrodenleitung vorgesehen ist, und dass der Leitungsadapter Lesemittel (321) zum Lesen der Elektrodenidentifikation, Sende- und Empfangsmittel (325) zu deren umkodierter Übermittlung in das Elektrostimulationsgerät sowie ein Sendemittel (323) zu deren Übermittlung an einen anordnungs-externen Empfänger aufweist.

2. Elektrostimulationsanordnung nach Anspruch 1, **dadurch gekennzeichnet, dass** der Leitungsadapter (220) im Lieferzustand zusammen mit dem Elektrostimulationsgerät (210) in einer gemeinsamen sterilen Verpackung angeordnet ist.

3. Elektrostimulationsanordnung nach Anspruch 1, **dadurch gekennzeichnet, dass** der Leitungsadapter im Lieferzustand zusammen mit der Elektrodenleitung in einer gemeinsamen sterilen Verpackung (250) angeordnet ist.

4. Elektrostimulationsanordnung nach einem der vorangehenden Ansprüche, **dadurch gekennzeichnet, dass** die auslesbare Elektrodenidentifikation (133; 333) mechanisch, optisch und/oder elektrisch, insbesondere elektromagnetisch oder mittels Hochfrequenz auslesbar ist und die Lesemittel des Leitungsadapters mechanische, optische und/oder elektrische Aufnahmemittel, insbesondere elektromagnetische Aufnahmemittel oder Hochfrequenz aufnehmende Mittel aufweisen.

5. Elektrostimulationsanordnung nach Anspruch 4, **dadurch gekennzeichnet, dass** die auslesbare Elektrodenidentifikation (133; 333) durch die Lesemittel des Leitungsadapters optisch berührungslos und/oder elektrisch galvanisch oder berührungslos auslesbar ist.

6. Elektrostimulationsanordnung nach Anspruch 4 oder 5, **dadurch gekennzeichnet, dass** die auslesbare Elektrodenidentifikation (133; 333) einen Barcode oder eine Buchstaben/Ziffern-Kombination, und die Lesemittel des Leitungsadapters einen Barcode-Scanner (321) oder einen Text-Scanner umfassen.

7. Elektrostimulationsanordnung nach Anspruch 6, **dadurch gekennzeichnet, dass** die optisch lesbare Elektrodenidentifikation (133; 333) an einem Stecker (231; 331) der Elektrodenleitung (130; 330) vorgesehen ist.

8. Elektrostimulationsanordnung nach einem der vorangehenden Ansprüche, **dadurch gekennzeichnet, dass** die Sendemittel (323; 325) des Leitungsadapters (320) eine Funkschnittstelle (323; 325) zur telemetrischen Übertragung der Elektrodenidentifikationsdaten in das Elektrostimulationsgerät oder zur drahtlosen Übermittlung derselben an eine korrespondierende Funkschnittstelle einer Therapiezentrale aufweisen.

9. Elektrostimulationsanordnung nach einem der vorangehenden Ansprüche, **dadurch gekennzeichnet, dass** der Leitungsadapter (320) einen Elektrodenidentifikations-Umsetzer (327) zur Umkodierung der gelesenen Elektrodenidentifikation gemäß Anschluss-Spezifikationen des Elektrostimulationsgeräts (310) und zur Übergabe der umkodierten Elektrodenidentifikation an die Sendemittel (325) zur Übermittlung in das Elektrostimulationsgerät aufweist.

10. Elektrostimulationsanordnung nach Anspruch 9, **dadurch gekennzeichnet, dass** Sendemittel (323) zur Übermittlung der Elektrodenidentifikation an den anordnungs-externen Empfänger zur Übersendung sowohl der gelesenen Elektrodenidentifikation als auch der umkodierten Elektrodenidentifikationsdaten ausgebildet sind.

11. Leitungsadapter (120; 220; 320) zum temporären Einsetzen zwischen das Elektrostimulationsgerät und die Elektrodenleitung in einer implantierbaren Elektrostimulationsanordnung (100; 300) nach einem der Ansprüche 1 bis 10, **dadurch gekennzeichnet, dass** er Lesemittel (321) zum Lesen der Elektrodenidentifikation, Sende- und Empfangsmittel (325) zu deren umkodierter Übermittlung in das Elektrostimulationsgerät (310) und Sendemittel (323) zu deren Übermittlung an einen anordnungs-externen Empfänger aufweist.

12. Leitungsadapter nach Anspruch 11, **dadurch gekennzeichnet, dass** er weiter einen Elektrodenidentifikations-Umsetzer (327) zur Umkodierung der gelesenen Elektrodenidentifikation gemäß Anschluss-Spezifikationen des Elektrostimulationsgeräts (310) und zur Übergabe der umkodierten Elektrodenidentifikation an die Sendemittel (325) zur Übermittlung in das Elektrostimulationsgerät aufweist.

13. Leitungsadapter nach Anspruch 11, **dadurch gekennzeichnet, dass** er einen Schrittmacherdatenspeicher (329) aufweist, der durch die Sende- und Empfangsmittel (325) vom Elektrostimulationsgerät (310) empfangene Schrittmacherdaten speichert.

14. Leitungsadapter nach Anspruch 12, **dadurch gekennzeichnet, dass** der Elektrodenidentifikations-Umsetzer (327) die Elektrodenidentifikation mit Hilfe der im Schrittmacherdatenspeicher (329) gespeicherten Schrittmacherdaten schrittmacherspezifisch umkodiert.

15. Leitungsadapter nach Anspruch 11, **dadurch gekennzeichnet, dass** er im Lieferzustand einzeln verpackt und insbesondere re-sterilisierbar ausgeführt ist.

## Claims

1. An implantable electrostimulation assembly (100; 300), comprising an electrostimulation device (110; 210; 310), a lead adapter (120; 220; 320), and an electrode lead (130; 330) that is connected to the electrostimulation device when in use,
wherein the electrode lead has a readable electrode identification (133; 333), **characterised in that**
the lead adapter (120; 220; 320) is intended for temporary insertion between the electrostimulation device and the electrode lead, and **in that** the lead adapter comprises read means (321) for reading the electrode identification, and transceiver means (325) for the re-coded transmission of the electrode identification into the electrostimulation device, and also a transmission means (323) for transmitting the electrode identification to an assembly-external receiver.

2. The electrostimulation assembly according to claim 1, **characterised in that** the lead adapter (220) is arranged together with the electrostimulation device (210) in a common sterile packaging for shipment.

3. The electrostimulation assembly according to claim 1, **characterised in that** the lead adapter is arranged together with the electrode lead in a common sterile packaging (250) for shipment.

4. The electrostimulation assembly according to any one of the preceding claims, **characterised in that** the readable electrode identification (133; 333) can be read mechanically, optically and/or electrically, in particular electromagnetically, or by means of high frequency, and the read means of the lead adapter have mechanical, optical and/or electrical pick-up means, in particular electromagnetic pick-up means or means for picking up high frequency.

5. The electrostimulation assembly according to claim 4, **characterised in that** the readable electrode identification (133; 333) is read out optically in a contactless manner and/or electrically in a galvanic or contactless manner by the read means of the lead adapter.

6. The electrostimulation assembly according to claim 4 or 5, **characterised in that** the readable electrode identification (133; 333) comprises a barcode or a letter/numeral combination, and the read means of the lead adapter comprise a barcode scanner (321) or a text scanner.

7. The electrostimulation assembly according to claim 6, **characterised in that** the optically readable electrode identification (133; 333) is provided on a connector (231; 331) of the electrode lead (130; 330).

8. The electrostimulation assembly according to any one of the preceding claims, **characterised in that** the transmission means (323; 325) of the lead adapter (320) comprise a radio interface (323; 325) for telemetrically transmitting the electrode identification data into the electrostimulation device or for wirelessly transmitting the electrode identification data to a corresponding radio interface of a treatment centre.

9. The electrostimulation assembly according to any one of the preceding claims, **characterised in that** the lead adapter (320) comprises an electrode identification converter (327) for re-coding the read electrode identification according to terminal specifications of the electrostimulation device (310) and for transferring the re-coded electrode identification to the transmission means (325) for transmission into the electrostimulation device.

10. The electrostimulation assembly according to claim 9, **characterised in that** the transmission means (323) for transmitting the electrode identification to the assembly-external receiver are designed to send both the read electrode identification and the re-coded electrode identification data.

11. A lead adapter (120; 220; 320) for temporary insertion between the electrostimulation device and the electrode lead in an implantable electrostimulation assembly (100; 300) according to any one of claims 1 to 10, **characterised in that** it comprises read means (321) for reading the electrode identification, and transceiver means (325) for the re-coded transmission of the electrode identification into the electrostimulation device (310), and transmission means (323) for transmitting the electrode identification to an assembly-external receiver.

12. The lead adapter according to claim 11, **characterised in that** it also comprises an electrode identification converter (327) for re-coding the read electrode identification according to terminal specifications of the electrostimulation device (310) and for transferring the re-coded electrode identification to the transmission means (325) for transmission into the electrostimulation device.

13. The lead adapter according to claim 11, **characterised in that** it comprises a pacemaker data memory (329), which stores pacemaker data received by the transceiver means (325) from the electrostimulation device (310).

14. The lead adapter according to claim 12, **characterised in that** the electrode identification converter (327) re-codes the electrode identification in a pacemakerspecific manner with the aid of the pacemaker data stored in the pacemaker data memory (329).

15. The lead adapter according to claim 11, **characterised in that** it is individually packaged for shipment and more particularly is designed so as to be re-sterilisable.

## Revendications

1. Agencement d'électrostimulation implantable (100; 300) avec un appareil d'électrostimulation (110 ; 210 ; 310), un adaptateur de ligne (120 ; 220 ; 320) et une ligne d'électrode (130 ; 330) raccordée à l'appareil d'électrostimulation dans l'état de fonctionnement,
où la ligne d'électrode présente une identification d'électrode (133 ; 333) lisible, **caractérisé en ce que**
l'adaptateur de ligne (120 ; 220 ; 320) est prévu pour une mise en place temporaire entre l'appareil d'électrostimulation et la ligne d'électrode, et que l'adaptateur de ligne présente des moyens de lecture (321) pour la lecture de l'identification d'électrode, des moyens d'émission et de réception (325) pour sa transmission dans l'appareil d'électrostimulation ainsi que des moyens d'émission (323) pour sa transmission à un récepteur externe à l'agencement.

2. Agencement d'électrostimulation selon la revendication 1, **caractérisé en ce que** l'adaptateur de ligne (220), à l'état de livraison, est disposé ensemble avec l'appareil d'électrostimulation (210) dans un emballage stérile commun.

3. Agencement d'électrostimulation selon la revendication 1, **caractérisé en ce que** l'adaptateur de ligne, à l'état de livraison, est disposé ensemble avec la ligne d'électrode dans un emballage stérile commun (250).

4. Agencement d'électrostimulation selon l'une des revendications précédentes, **caractérisé en ce que** l'identification d'électrode (133 ; 333) lisible est lisible mécaniquement, optiquement et/ou électriquement, notamment électromagnétiquement ou au moyen d'une haute fréquence et les moyens de lecture de l'adaptateur de ligne présentent des moyens d'enregistrement mécaniques, optiques et/ou électriques, notamment des moyens d'enregistrement électromagnétiques ou des moyens d'enregistrement de haute fréquence.

5. Agencement d'électrostimulation selon la revendication 4, **caractérisé en ce que** l'identification d'électrode (133 ; 333) lisible est lisible par les moyens de lecture de l'adaptateur de ligne de manière optique sans contact, et/ou électriquement de manière galvanique ou sans contact.

6. Agencement d'électrostimulation selon la revendication 4 ou 5, **caractérisé en ce que** l'identification d'électrode (133 ; 333) lisible comprend un code-barres ou une combinaison lettres/chiffres, et les moyens de lecture de l'adaptateur de ligne comprennent un lecteur de code-barres (321) ou un lecteur de texte.

7. Agencement d'électrostimulation selon la revendication 6, **caractérisé en ce que** l'identification d'électrode (133 ; 333) lisible optiquement est prévue sur une prise (231 ; 331) de la ligne d'électrode (130 ; 330).

8. Agencement d'électrostimulation selon l'une des revendications précédentes, **caractérisé en ce que** les moyens d'émission (323 ; 325) de l'adaptateur de ligne (320) présentent une interface radio (323 ; 325) pour la transmission télémétrique des données d'identification d'électrode dans l'appareil d'électrostimulation ou pour la transmission sans fil de celles-ci vers une interface radio correspondante d'une centrale de thérapie.

9. Agencement d'électrostimulation selon l'une des revendications précédentes, **caractérisé en ce que** l'adaptateur de ligne (320) présente un convertisseur d'identification d'électrodes (327) pour la conversion des codes de l'identification d'électrode lue selon des spécifications de raccordement de l'appareil d'électrostimulation (310) et pour la transmission de l'identification d'électrode à code converti vers les moyens d'émission (325) pour la transmission dans l'appareil d'électrostimulation.

10. Agencement d'électrostimulation selon la revendication 9, **caractérisé en ce que** des moyens d'émission (323) sont conçus pour la transmission de l'identification d'électrode vers le récepteur externe à l'agencement pour la transmission à la fois de l'identification d'électrode lue et des données d'identification d'électrode à code converti.

11. Adaptateur de ligne (120; 220; 320) pour la mise en place temporaire entre l'appareil d'électrostimulation et la ligne d'électrode dans un agencement d'électrostimulation (100 ; 300) implantable selon l'une des revendications 1 à 10, **caractérisé en ce qu'**il présente des moyens de lecture (321) pour la lecture de l'identification d'électrode, des moyens d'émission er de réception (325) pour sa transmission à codage converti dans l'appareil d'électrostimulation (310) et des moyens d'émission (323) pour sa transmission vers un récepteur externe à l'agencement.

12. Adaptateur de ligne selon la revendication 11, **caractérisé en ce qu'**il présente en outre un convertisseur d'identification d'électrode (327) pour la conversion de code de l'identification d'électrode lue selon des spécifications de raccordement de l'appareil d'électrostimulation (310) et pour la transmission de l'identification d'électrode à codage converti vers les moyens d'émission (325) pour la transmission dans l'appareil d'électrostimulation.

13. Adaptateur de ligne selon la revendication 11, **caractérisé en ce qu'**il présente une mémoire de données de stimulateur cardiaque (329), qui stocke les données du stimulateur cardiaque reçues de l'appareil d'électrostimulation (310) par les moyens d'émission et de réception (325).

14. Adaptateur de ligne selon la revendication 12, caractérisé en ce le convertisseur d'identification d'électrode (327) convertit le code de l'identification d'électrode à l'aide des données de stimulateur cardiaque stockées dans la mémoire de données de stimulateur cardiaque (329) de manière spécifique au stimulateur cardiaque.

15. Adaptateur de ligne selon la revendication 11, **caractérisé en ce que**, dans l'état de livraison, il est emballé individuellement et est notamment conçu pour être stérilisé plusieurs fois.
